# EUROPEAN PATENT SPECIFICATION

(11) **EP 2 473 123 B1**
(45) Date of publication and mention of the grant of the patent: **30.01.2019**
(21) Application number: 10749738.0
(22) Date of filing: 27.08.2010
(51) Int. Cl.: A61B 17/3207, A61M 25/00, A61B 17/34, A61B 17/02

(54) **LANCET MICRO-CATHETER**
MIKROKATHETER MIT LANZETTE
MICRO-CATHÉTER À LANCETTE

(30) Priority: 03.09.2009 US 275795 P
(43) Date of publication of application: 11.07.2012
(73) Proprietor: Boston Scientific Limited, Hamilton HM11 (BM)
(72) Inventor: ROTTENBERG, Dan, 34601 Haifa (IL); ZAKAY, Abi, 30900 Zichron Yacov (IL); SACHER, Ron, 46702 Herzelia (IL)
(74) Representative: Vossius & Partner Patentanwälte Rechtsanwälte mbB
(86) International application number: PCT/US2010/046880
(87) International publication number: WO 2011/028632

(56) References cited:
- WO-A1-2010/027843
- WO-A2-2007/115314
- US-A1- 2004 147 903
- US-A1- 2004 147 903
- US-A1- 2005 149 062
- US-A1- 2006 074 442
- US-B2- 7 320 695

## Description

### FIELD OF THE INVENTION

The present invention relates to a micro-catheter having a sharp lancet tip at its distal end, useful, for example, to penetrate hard-calcified tissue and plaque in a vessel.

### BACKGROUND OF THE INVENTION

Chronic Total Occlusion (CTO) is an arterial vessel blockage that impedes proper blood flow beyond the obstruction. Poor blood flow can lead to insufficient delivery of blood to muscles and vital organs. CTO can occur both in the coronary and peripheral arteries, resulting in disability and even death. The underlying cause of CTO is atherosclerosis.

In order to reverse the effects of CTO, proper blood flow must be reestablished. This must be achieved by either driving through and removing the CTO or creating a bypass around the CTO. Under either scenario, the first step is to "cross" or drive a guidewire through or around the CTO. If the occlusion is relatively new, the plaque is likely to be soft and the guidewire will penetrate and cross the plaque. Thereafter, balloon angioplasty and stenting can be performed. However, if the plaque has been lodged in the vessel for several weeks or months, the plaque can become much harder as the occlusion becomes fibrotic and calcified, making it almost impossible for a guidewire to cross. Failure to cross the obstruction is the primary failure mode for CTO recanalization, and often leads to the abortion of the interventional procedure in favor of a surgical bypass procedure instead, with higher costs and complications.

It is known in the art that when trying to cross an occluded blood vessel with a guidewire, in many cases the guidewire inadvertently deflects into the subintimal space between the intimal layer and the adventitial layer of the blood vessel. Once in the subintimal space, the guidewire can be advanced along side the CTO (which is on the other side of the intimal wall) and beyond the length of the occlusion. Dissection of the blood vessel and guidewire insertion into subintimal space is very common, especially in blocked peripheral blood vessels. The difficulty in many cases is to direct the guidewire back into the blood vessel's true lumen beyond the occlusion so that this new channel can be dilated and used as an internal conduit for blood supply. Known as subintimal recanalization, this procedure can be very useful. There are some advantages to passing around the occlusion, rather than punching directly through it. The subintimal space is more likely to produce a smooth surface for blood flow versus the original lumen, which has remnants of the calcified plaque.

The technique has limited acceptance due to technical challenges of the procedure. Among those who perform subintimal recanalization, there is a technical failure rate of up to 30% due to the inability to reenter the distal true lumen at the specific desired location. Therefore, most clinicians will first try to cross the occlusion. If unsuccessful and if they enter the subintimal space, they will try to reenter the true lumen. If they cannot reenter after several minutes, they will abandon the case and schedule the patient for surgery.

During percutaneous transluminal recanalization, a variety of guidewires are used to pass the occlusion. If the occlusion is hard, a stiff wire is used to cross. Usually the proximal cap of the occlusion is the harder section, and if crossed, then crossing the rest of the occlusion is relative easy.

If during percutaneous extraluminal recanalization via the subintimal approach the true lumen cannot be reentered with guidewire manipulation, a true lumen reentry device must be used. Currently there are two specially designed reentry devices on the market.

US2005/0149062A1 discloses a micro-catheter comprising a hypo-tube comprising a proximal section and a distal section and a sharp tip formed at the distal end of the distal section as well as a plurality of slots or shaped cuts in the proximal section.

Published patent applications US20030236542, WO2006105244, US2003120195, and US2006094930 and US Patent 6081738 all describe reentry devices and methods, based on curved needle penetration, used with some type of rotational imaging system.

PCT patent application PCT/IL2008/000449 describes a reentry balloon catheter that eliminates the needs for rotational orientation and operator direction via imaging guidance. This balloon catheter automatically bends its distal end in the direction of the true lumen. This device eliminates the need for a curved needle, and instead allows direct penetration with or without the support of a straight guidewire or hypotube.

### SUMMARY OF THE INVENTION

The present invention seeks to provide a micro-catheter having sharp lancet tip at its distal end, as is described further in detail hereinbelow. The micro-catheter design provides both high flexibility and high torque-ability, despite its small diameter. The distal-end section is hard and sharp, designed to penetrate hard-calcified tissue and plaque in the vessel.

The micro-catheter can be used during CTO (Chronic Total Occlusion) percutaneous transluminal recanalization when conventional guidewires cannot pass the occlusion. Usually the proximal fibrous cap of the occlusion can be penetrated with the hard lancet tip of this new micro-catheter, and smaller guidewires can be inserted through the micro-catheter's internal lumen to penetrate the rest of the occlusion. If the plaque is hard along the entire length of the occlusion, the micro-catheter can be used for full CTO penetration, and replaced by a guide-wire afterwards.

A balloon catheter, preferably with a distal tip-less inverted neck balloon, can be used to stabilize and adjust the micro-catheter lancet tip to the center of the occlusion proximal cap. With the tip-less balloon catheter, the balloon catheter can be advanced all the way to the CTO plaque cap and then inflated. The micro-catheter is centered and can be advanced directly towards the plaque for puncturing the cap and crossing the CTO.

If a subintimal approach is used and the true lumen cannot be reentered with a guidewire alone, the lancet micro-catheter can be used to re-enter the true lumen,. The micro-catheter of the invention is pushed over the guidewire distal to the occlusion, and the lancet tip, which can be manually bent, is used to penetrate the plaque and re-enter the true lumen. If a reentry balloon catheter is used, the lancet micro-catheter is pushed through the balloon catheter lumen to reach the reentry point, then it exits the balloon in the direction of the true lumen, and penetrates the intimal wall and re-enter the true lumen.

The micro-catheter of the invention has good pushability to apply axial penetration force through the lancet tip, has good torque-ability to allow lancet tip rotation for better penetration at sharp plaque approach angles, and has good flexibility to safely navigate the blood vessel curves. However, it is not simple or obvious to achieve all these features in the same device. For example, the properties of good torque-ability and good flexibility generally contradict one another.

To accomplish all above demands, the micro-catheter of the invention is made from stainless steel or nitinol or other biocompatible metals for making a hypo-tube. The hypo-tube is laser cut, or mechanically cut, in such profiles that will provide required flexibility, torque-ability and push-ability. It is noted that standard spiral (e.g., spring shape) cutting does not provide all the above demands; instead it provides only high flexibility with very low torque-ability and push-ability.

The distal section of the hypo-tube preferably remains uncut for better strength and superior penetration, and the tip is cut in sharp formation, preferably in lancet tip shape. Other shapes of sharp tips can also be used.

The use of such metal hypo-tubes provide the flexible section and the hard sharp tip made from same tube and material, reducing the device wall-thickness and avoiding the need of connecting two elements (e.g., a metal needle tip to a flexible plastic tube).

The inner and/or external surfaces of the hypo-tube can be partially or fully covered by a low friction jacket, like polyether block amide (e.g., PEBAX) or PTFE (polytetrafluoroethylene), to keep the micro-catheter surfaces smooth and without slices, spurs, burrs or notches.

### BRIEF DESCRIPTION OF THE DRAWINGS

The present invention will be understood and appreciated more fully from the following detailed description, taken in conjunction with the drawings in which:
Fig. 1 is a simplified illustration of a micro-catheter, constructed and operative in accordance with an embodiment of the present invention;
Fig. 2 is a simplified illustration of the micro-catheter penetrating a proximal cap of an occlusion with a lancet tip, in accordance with an embodiment of the present invention;
Fig. 3 is a simplified illustration of the micro-catheter in a blood vessel subintimal space, inside a reentry balloon, reentering the true lumen distal to the occlusion, in accordance with an embodiment of the present invention;
Figs. 4A and 4B are simplified illustrations of the micro-catheter lancet tip penetrating plaque in different tip directions;
Fig. 5 is a partial sectional simplified illustration of a catheter hypo-tube laser cut profile, in accordance with an embodiment of the present invention;
Fig. 6 is another partial sectional simplified illustration of the catheter hypo-tube laser cut profile, having different pitch and cutting sectors / angles;
Fig. 7 is a simplified illustration of a micro-catheter, constructed and operative in accordance with another embodiment of the present invention, including a double needle tip; and
Fig. 8 is a simplified illustration of micro-catheter, constructed and operative in accordance with yet another embodiment of the present invention, including a side-port needle.

### DETAILED DESCRIPTION OF EMBODIMENTS

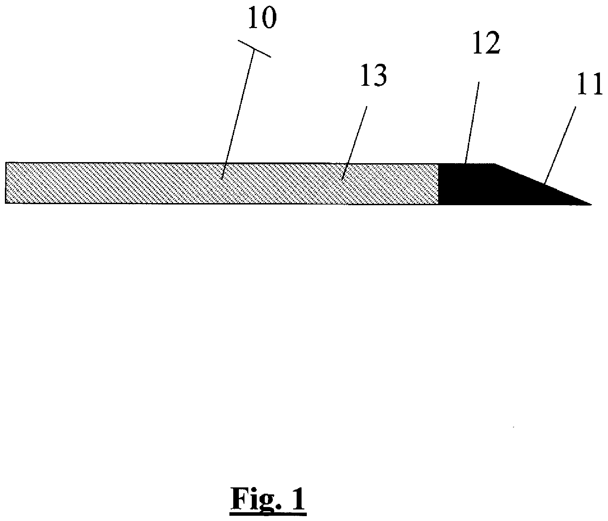

Reference is now made to Fig. 1, which illustrates a micro-catheter 10, constructed and operative in accordance with a non-limiting embodiment of the present invention.

The micro-catheter 10 has a lancet tip 11 at a distal end of a distal section 12, and may be constructed with a hollow lumen through its length. The micro-catheter design provides high flexibility, high pushability and high torqueability, despite its small diameter. The distal end section is hard and sharp, designed to penetrate hard and calcified tissues.

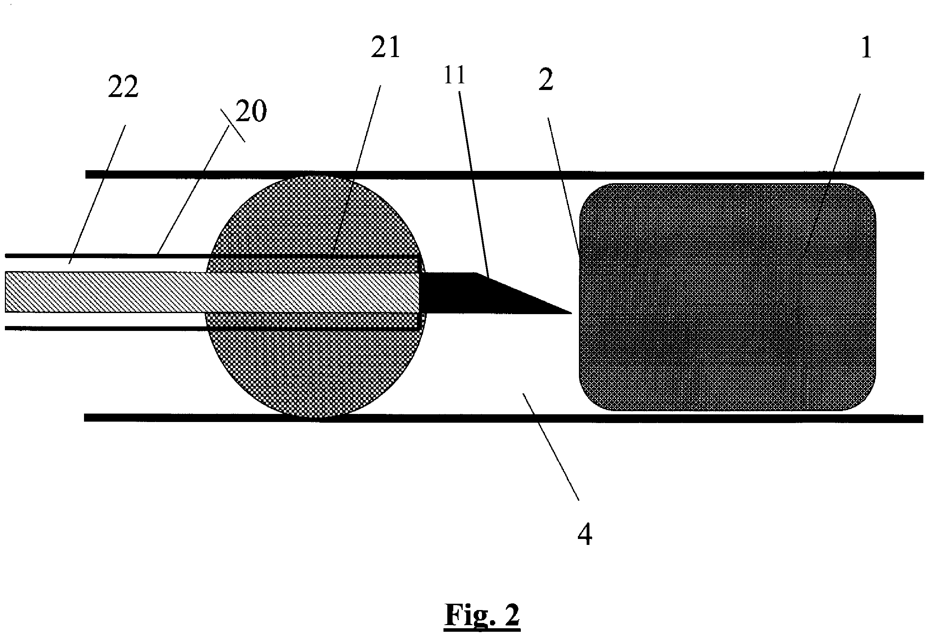

Reference is now made to Fig. 2. The micro-catheter 10 can be used during CTO percutaneous transluminal recanalization when conventional guide-wires cannot pass an occlusion 1. Usually a proximal cap 2 of the occlusion 1 is penetrated with the hard lancet tip 11, and guide-wires are inserted through the micro-catheter hollow lumen to penetrate the rest of the occlusion. Sometimes the plaque within the diseased blood vessel is hard over the entire length, and micro-catheter 10 can be used for full occlusion penetration, replaced by a guide-wire afterwards.

The micro-catheter 10 can be employed together with a balloon catheter 20, which preferably has a distal tip-less inverted neck balloon 21 (as described in PCT patent application PCT/IL2008/000449). The micro-catheter 10 can be inserted in a balloon catheter lumen 22 of balloon catheter 20, thereby placing the lancet tip very close to the occlusion. This arrangement can be used to stabilize and adjust micro-catheter 10 and its lancet tip 11 to the center of the proximal cap 2.

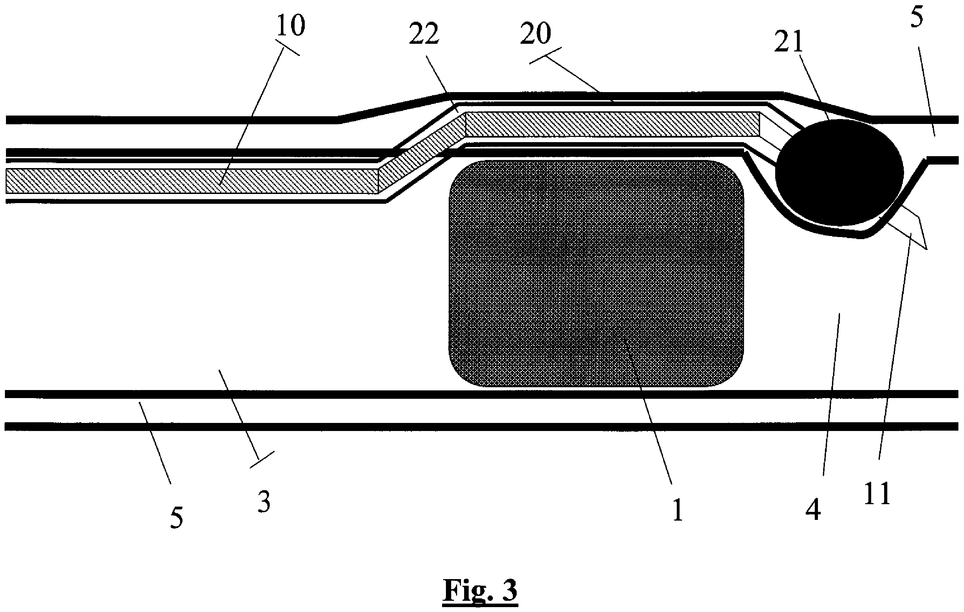

Reference is now made to Fig. 3. If a subintimal approach is used, the lancet micro-catheter 10 can be used if during percutaneous extraluminal recanalization the true lumen 4 of a vessel 3 cannot be reentered with the guidewire. The micro-catheter 10 is pushed over the guidewire in the subintimal space 5, distal to the occlusion, and the lancet tip 11 is used to penetrate the plaque and re-enter the true lumen. The micro-catheter 10 can be pushed through the balloon catheter lumen 22 to reach the reentry point, protrude from balloon 21 in the direction of the true lumen 4, and re-enter into the true lumen 4 of the vessel 3.

A proximal section 13 of micro-catheter 10 can be slightly bent such as by the user, by applying a bending force on the hypo-tube cut section, so as to convert the micro-catheter 10 from a straight configuration to an angled configuration at the junction of section 13 and distal section 12.

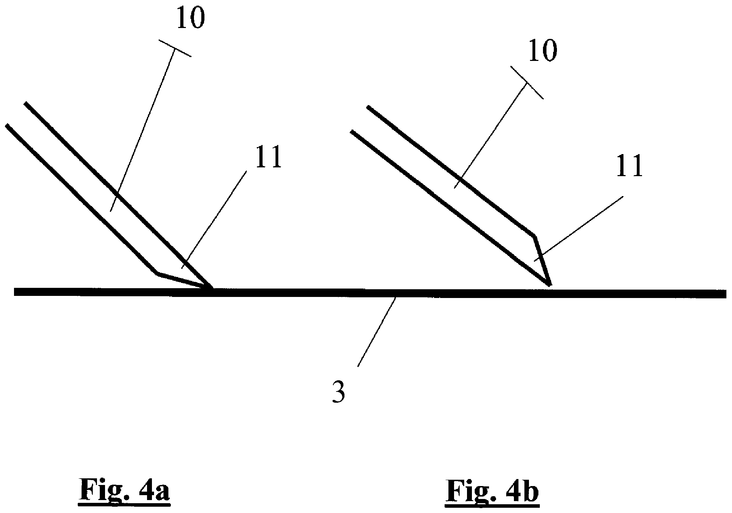

The micro-catheter 10 should have good pushability to apply axial penetration force through lancet tip 11. At the same time, micro-catheter 10 needs to have good torqueability to allow rotating lancet tip 11 for better plaque penetration using different sharp angle approaches (as seen in Figs. 4A and 4B). Yet the micro-catheter 10 also needs to have good flexibility to pass through the blood vessel curvature.

To accomplish all the above demands, some of which contradict one other (e.g., good torqueability and good flexibility), the micro-catheter 10 is laser cut or mechanically cut from a hypo-tube made from stainless steel or nitinol or other biocompatible metal in a manner now described that provides the required flexibility, torqueability and pushability.

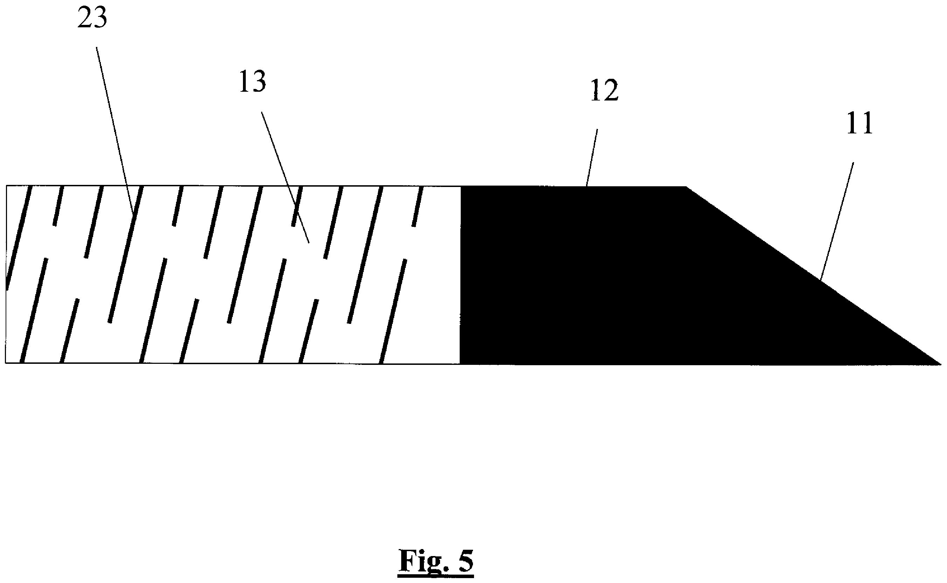

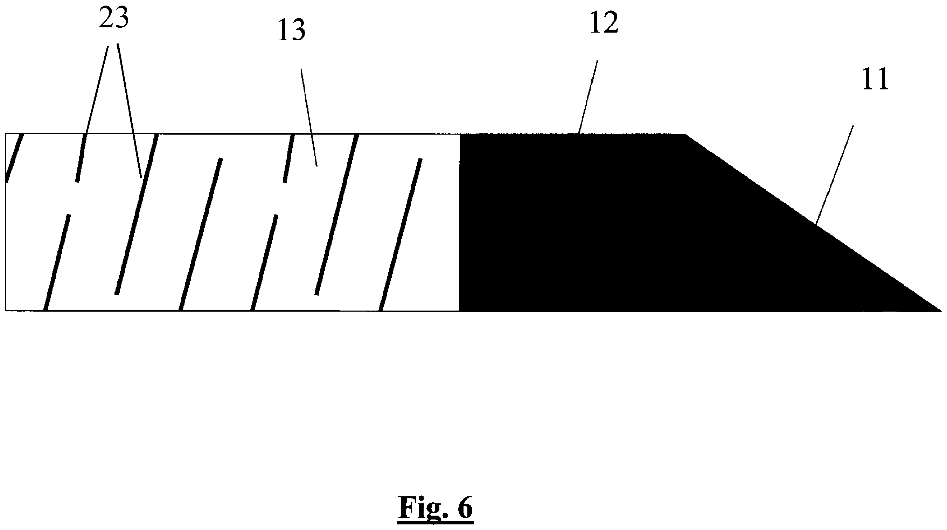

Reference is now made to Figs. 5 and 6. In accordance with an embodiment of the invention, the proximal section 13 of micro-catheter 10 is formed with a plurality of cuts 23 which are not perpendicular to a longitudinal axis of the proximal section 13 of micro-catheter 10. The cuts 23 are made partially around the circumference of the proximal section 13 of micro-catheter 10 with uncut portions separating the cuts 23 from one another. In one embodiment, cuts 23 subtend a larger angle, called a cut sector angle, than the uncut portions, called an uncut sector angle. In one example, the cut sector angle is greater than or equal to 90°, and the uncut portions subtend an angle less than 90°.

The cuts 23 can be formed by intermittent cutting of the hypo-tube section 13, while rotating and advancing it. The metal hypo-tube is rotated at a desired rotational speed, while the hypo-tube and the cutting tool are moved axially relative to each other, thus providing the cutting pitch. The cutting tool, such as a laser or mechanical cutting tool, cuts the tube intermittently during every full, 360° rotation.

For example, the pitch can be 0.2-2.0 mm, and cut sector angles are 245°, and the uncut sectors are 45°. Alternatively, the metal micro-tube is slotted in intervals between 90° and 230°, and unslotted between 5° and 90° for most of its length to achieve high flexibility, torqueability, and pushability. Other angles and pitches can be used to carry out the invention.

In accordance with an embodiment of the invention, the sum of the cut sector and uncut sector angles is less than 360°, providing phase shift of the cut and uncut profile every full (360°) rotation. This provides basically the same micro-catheter characteristics in all bending / pushing directions.

The larger the cut sector, relative to the un-cut sector, the more flexible and less torqueable and pushable is the micro-catheter 10. The smaller the pitch, the flexibility increases, but the torqueability is reduced.

Micro-catheter 10 can be divided to segments having different characteristics by changing the pitch or cutting sector angles.

The distal section 12 of the hypo-tube preferably remains uncut for better strength and superior penetration, and the tip is cut in a sharp formation, preferably in a lancet tip shape.

The length of the distal section 12 is preferably short (shorter than proximal section 13), usually less then 10 mm, to allow distal section 12 and lancet tip 11 to easily pass through blood vessel curvatures, over a guidewire or inside balloon catheter 20.

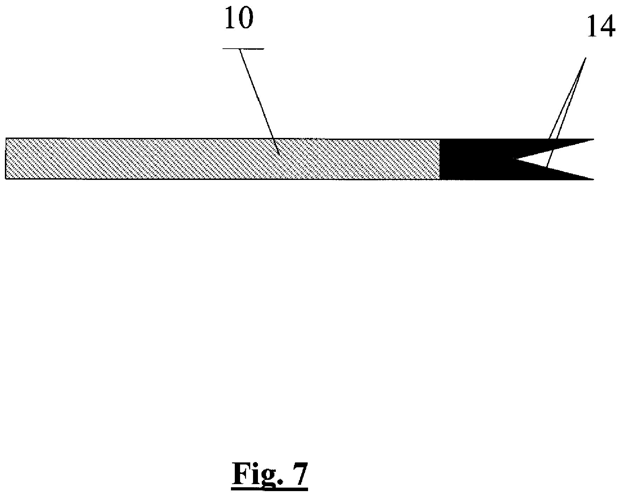

Other shapes of sharp tips 11 can also be used. In another embodiment of the invention, the hypo-tube tip is cut to have two parallel sharp edges 14 (Fig. 7).

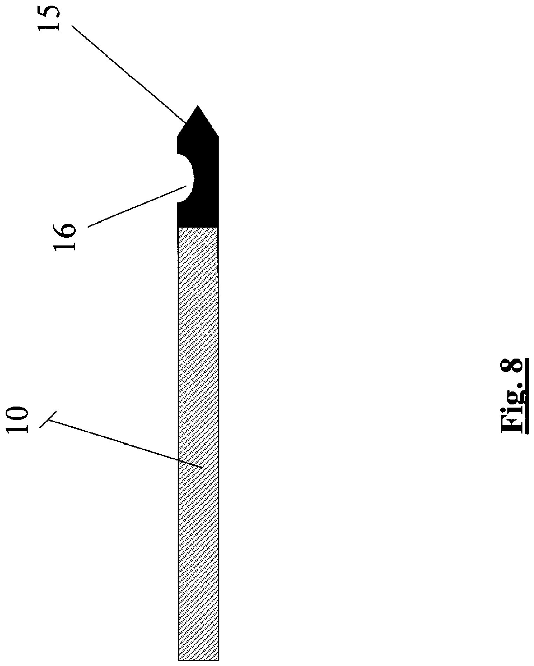

In another embodiment of the invention, the hypo-tube tip is shaped as a closed sharp tip 15, having a side port 16 that can serve as an exit port for a guidewire (Fig. 8).

The proximal end of the hypo-tube section 13 is preferably uncut for better user handling and easy connection of luer connectors. It is noted that using the same material to make the micro-catheter 10 and its sharp tip 11 can reduce the device wall-thickness and avoids having to connect a metal needle tip to a flexible plastic tube.

The inner and/or external surfaces of the hypo-tube can be completely or partially covered by a low friction covering or jacket, such as but not limited to PEBAX or PTFE, to keep the surfaces smooth and without slices, spurs, burrs or notches.

## Claims

1. A micro-catheter (10) comprising:
a hypo-tube comprising a proximal section (13) and a distal section (12); and
a sharp tip (11) formed at a distal end of said distal section (12);
**characterized in that** said proximal section (13) of said micro-catheter (10) is formed with a plurality of cuts (23) which are not perpendicular to a longitudinal axis of said proximal section (13), said cuts (23) being made partially and intermittently around a circumference of said proximal section (13) for most of the length of the proximal section (13) with uncut portions separating said cuts (23) from one another.

2. The micro-catheter (10) according to claim 1, wherein said cuts (23) subtend a larger angle, called a cut sector angle, than the uncut portions, called an uncut sector angle.

3. The micro-catheter (10) according to claim 2, wherein said cut sector angle is greater than or equal to 90°, and the uncut sector angle is less than 90°.

4. The micro-catheter (10) according to claim 1, wherein said hypo-tube has a hollow lumen through its length.

5. The micro-catheter (10) according to claim 1, wherein a pitch between said cuts (23) is between 0.2-2.0 mm.

6. The micro-catheter (10) according to claim 2, wherein a sum of the cut sector and uncut sector angles is such that there is a phase shift of cut and uncut portions every rotation.

7. The micro-catheter (10) according to claim 1, wherein said distal section (12) is left uncut.

8. The micro-catheter (10) according to claim 1, wherein said distal section (12) is shorter than said proximal section (13).

9. The micro-catheter (10) according to claim 1, wherein said sharp tip (11) comprises a lancet tip (11).

10. The micro-catheter (10) according to claim 1, wherein said sharp tip (11) comprises two parallel sharp edges (14).

11. The micro-catheter (10) according to claim 1, wherein said sharp tip (15) comprises a side port (16).

12. The micro-catheter (10) according to claim 1, wherein surfaces of said hypo-tube are at least partially covered by a low friction covering.

## Patentansprüche

1. Mikrokatheter (10) mit:
einem Hyporohr, das einen proximalen Abschnitt (13) und einen distalen Abschnitt (12) aufweist; und
einer scharfen Spitze (11), die an einem distalen Ende des distalen Abschnitts (12) ausgebildet ist;
**dadurch gekennzeichnet, dass** der proximale Abschnitt (13) des Mikrokatheters (10) mit einer Vielzahl von Einschnitten (23) ausgebildet ist, die nicht senkrecht zu einer Längsachse des proximalen Abschnitts (13) sind, wobei die Einschnitte (23) teilweise und intermittierend um einen Umfang des proximalen Abschnitts (13) über den größten Teil der Länge des proximalen Abschnitts (13) ausgeführt sind, wobei nicht eingeschnittene Abschnitte die Einschnitte (23) voneinander trennen.

2. Mikrokatheter (10) nach Anspruch 1, wobei die Einschnitte (23) einen größeren Winkel, der als Winkel des eingeschnittenen Sektors bezeichnet wird, einschließen als die nicht eingeschnittenen Abschnitte, der als ein Winkel des nicht eingeschnittenen Sektors bezeichnet wird.

3. Mikrokatheter (10) nach Anspruch 2, wobei der Winkel des eingeschnittenen Sektors größer oder gleich 90° ist, und der Winkel des nicht eingeschnittenen Sektors kleiner als 90° ist.

4. Mikrokatheter (10) nach Anspruch 1, wobei das Hyporohr ein hohles Lumen durch seine Länge aufweist.

5. Mikrokatheter (10) nach Anspruch 1, wobei ein Abstand zwischen den Einschnitten (23) zwischen 0,2-2,0 mm beträgt.

6. Mikrokatheter (10) nach Anspruch 2, wobei eine Summe der Winkel des eingeschnittenen Sektors und des nicht eingeschnittenen Sektors so gestaltet ist, dass es eine Phasenverschiebung der eingeschnittenen und nicht eingeschnittenen Abschnitte bei jeder Umdrehung gibt.

7. Mikrokatheter (10) nach Anspruch 1, wobei der distale Abschnitt (12) nicht eingeschnitten belassen wird.

8. Mikrokatheter (10) nach Anspruch 1, wobei der distale Abschnitt (12) kürzer als der proximale Abschnitt (13) ist.

9. Mikrokatheter (10) nach Anspruch 1, wobei die scharfe Spitze (11) eine Lanzettenspitze (11) aufweist.

10. Mikrokatheter (10) nach Anspruch 1, wobei die scharfe Spitze (11) zwei parallele scharfe Kanten (14) aufweist.

11. Mikrokatheter (10) nach Anspruch 1, wobei die scharfe Spitze (15) eine seitliche Öffnung (16) Aufweist.

12. Mikrokatheter (10) nach Anspruch 1, wobei Oberflächen des Hyporohrs mindestens teilweise durch eine reibungsarme Bedeckung bedeckt sind.

## Revendications

1. Microcathéter (10) comprenant :
un hypotube comprenant une section proximale (13) et une section distale (12) ; et
une pointe acérée (11) formée à une extrémité distale de ladite section distale (12) ;
**caractérisé en ce que** ladite section proximale (13) dudit microcathéter (10) est formée avec une pluralité d'entailles (23) qui ne sont pas perpendiculaires à un axe longitudinal de ladite section proximale (13), lesdites entailles (23) étant faites partiellement et de manière intermittente autour d'une circonférence de ladite section proximale (13) pour la plus grande partie de la longueur de la section proximale (13) avec des parties non entaillées séparant lesdites entailles (23) les unes des autres.

2. Microcathéter (10) selon la revendication 1, où lesdites entailles (23) sous-tendent un angle plus grand, appelé angle de secteur entaillé, que les parties non entaillées, appelé angle de secteur non entaillé.

3. Microcathéter (10) selon la revendication 2, où ledit angle de secteur entaillé est plus grand que ou égal à 90°, et l'angle de secteur non entaillé est moins grand que 90°.

4. Microcathéter (10) selon la revendication 1, où ledit hypotube a une lumière creuse sur sa longueur.

5. Microcathéter (10) selon la revendication 1, où un pas entre lesdites entailles (23) est entre 0,2-2,0 mm.

6. Microcathéter (10) selon la revendication 2, où une somme des angles de secteur entaillé et de secteur non entaillé est telle qu'il y a un déphasage des parties entaillées et non entaillées à chaque rotation.

7. Microcathéter (10) selon la revendication 1, où ladite section distale (12) est laissée non entaillée.

8. Microcathéter (10) selon la revendication 1, où ladite section distale (12) est plus courte que ladite section proximale (13).

9. Microcathéter (10) selon la revendication 1, où ladite pointe acérée (11) comprend une pointe de lancette (11).

10. Microcathéter (10) selon la revendication 1, où ladite pointe acérée (11) comprend deux bords acérés parallèles (14).

11. Microcathéter (10) selon la revendication 1, où ladite pointe acérée (15) comprend un orifice latéral (16).

12. Microcathéter (10) selon la revendication 1, où les surfaces dudit hypotube sont revêtues au moins en partie d'un revêtement à faible frottement.
